# EUROPEAN PATENT APPLICATION

(11) **EP 4 835 054 A1**
(43) Date of publication of application: **04.11.2026**
(21) Application number: 24913126.9
(22) Date of filing: 27.12.2024
(51) Int. Cl.: C12N 5/071, C12N 5/077

(54) **METHOD FOR PRODUCING CELL STRUCTURE**

(30) Priority: 28.12.2023 JP 2023223154
(71) Applicant: Toppan Holdings Inc., Tokyo 110-0016 (JP)
(72) Inventor: HIRAOKA, Yasuyuki, Tokyo 110-0016 (JP); HATTORI, Koichi, Tokyo 110-0016 (JP)
(74) Representative: Plasseraud IP
(86) International application number: PCT/JP2024/046444
(87) International publication number: WO 2025/143240

(57) **Abstract**

The present invention relates to a method for producing a cell structure having a vascular structure, the method including: a gelation step of gelling a cell-containing solution containing cells including vascular endothelial cells to form a gel containing the cells; and a culture step of culturing the cells in the gel, wherein the gel containing the cells is formed by bringing a plurality of gelling agents into contact with a gelling promoter.

## Description

### [Technical Field]

The present invention relates to a method for producing a cell structure.

### [Background Art]

In pharmaceutical processes, when evaluating the vascular toxicity of compounds such as drugs, planar cultured cells or animals such as mice are typically used. However, since the occurrence mechanisms of side effects such as vascular toxicity are complicated and diverse, it is difficult to accurately evaluate the toxicity of compounds by toxicity assessment methods using planar cultured cells. Animal studies using mice may also be difficult to use for risk assessment from the perspective of differences between biological species and social situations such as animal protection. In order to solve these problems, development of artificial tissues having vascular structures using human-derived cells is currently underway (for example, PTL 1).

### [Citation List]

### [Patent Literature]

PTL 1: WO 2021-100709 A

### [Summary of Invention]

### [Technical Problem]

The inventors of the present invention have developed a method for constructing a cell structure having a vascular structure by embedding cells in a gel (hydrogel) and then culturing the cells (PTL 1). The inventors of the present invention have newly found that this conventional cell structure has a problem that the angiogenic ability may vary even if the tissue is produced by the same method and on the same day (instability of angiogenic ability).

The present invention has been made to provide a method for producing a cell structure that can suppress variations in angiogenic ability.

### [Solution to Problem]

An embodiment of the present invention is as follows.
[1] A method for producing a cell structure having a vascular structure, the method including:
   a gelation step of gelling a cell-containing solution containing cells including vascular endothelial cells to form a gel containing the cells; and
   a culture step of culturing the cells in the gel, wherein
   the gel containing the cells is formed by bringing a plurality of gelling agents into contact with a gelling promoter.
[2] The method according to [1], wherein the cell-containing solution further contains an extracellular matrix component and a polyelectrolyte.
[3] The method according to [2], wherein the extracellular matrix component includes collagen.
[4] The method according to [2], wherein the polyelectrolyte includes heparin.
[5] The method according to any of [1] to [4], wherein the plurality of gelling agents include fibrinogen and sodium alginate.
[6] The method according to any of [1] to [5], wherein the gelling promoter includes thrombin and calcium chloride.
[7] The method according to any of [1] to [6], wherein the gelation step is a step of forming the gel by injecting the cell-containing solution containing the cells and the gelling promoter into a droplet of a reaction liquid containing the plurality of gelling agents.
[8] The method according to [7], wherein a volume ratio of a droplet amount of the reaction liquid to an injection amount of the cell-containing solution is 2 to 10.
[9] The method according to any of [2] to [4], further including, prior to the gelation step, a step of mixing the cells, the extracellular matrix component and the polyelectrolyte to obtain a mixed solution.
[10] The method according to [9], further including, prior to the gelation step, a step of centrifuging the mixed solution to obtain a precipitate containing the cells, the extracellular matrix component and the polyelectrolyte.
[11] The method according to any of [2] to [4], [9] and [10], wherein a concentration of the polyelectrolyte is greater than 0 mg/mL and 1.5 mg/mL or less relative to a total amount of the cell-containing solution.
[12] The method according to any of [2] to [4] and [9] to [11], wherein a concentration of the extracellular matrix component is greater than 0 mg/mL and 1.5 mg/mL or less relative to a total amount of the cell-containing solution.
[13] The method according to any of [1] to [12], wherein the cells further include hepatocytes.
[14] The method according to any of [1] to [13], wherein the cells further include hepatic stellate cells.
[15] The method according to any of [1] to [14], wherein a ratio of the number of vascular endothelial cells to the total number of cells is 5% or greater and 50% or less.
[16] A method for producing a cell structure having a vascular structure, the method including:
   a step of mixing cells including vascular endothelial cells, an extracellular matrix component and a polyelectrolyte to obtain a mixed solution;
   a step of centrifuging the mixed solution to obtain a precipitate containing the cells, the extracellular matrix component and the polyelectrolyte;
   a step of obtaining a cell-containing solution containing the precipitate and a gelling promoter;
   a step of forming a gel by injecting the cell-containing solution into a droplet of a reaction liquid containing a plurality of gelling agents; and
   a culture step of culturing the cells in the gel, wherein
   a concentration of the polyelectrolyte is greater than 0 mg/mL and 1.5 mg/mL or less relative to a total amount of the cell-containing solution,
   a concentration of the extracellular matrix component is greater than 0 mg/mL and 1.5 mg/mL or less relative to a total amount of the cell-containing solution,
   the plurality of gelling agents include fibrinogen and sodium alginate,
   a content of the fibrinogen is 1 mg/ml to 10 mg/ml relative to a total amount of the reaction liquid,
   a content of the sodium alginate is 0.01 mass% to 2 mass% relative to a total amount of the reaction liquid,
   the gelling promoter includes thrombin and calcium ions,
   a content of the thrombin is 10 unit/ml to 30 unit/ml relative to a total amount of the cell-containing solution,
   a content of the calcium ions is 1 mM to 10 mM relative to a total amount of the cell-containing solution, and
   a volume ratio of a droplet amount of the reaction liquid to an injection amount of the cell-containing solution is 2 to 10.
[17] A method for producing a cell structure having a vascular structure, the method including:
   a step of mixing cells including vascular endothelial cells, an extracellular matrix component and a polyelectrolyte to obtain a mixed solution;
   a step of centrifuging the mixed solution to obtain a precipitate containing the cells, the extracellular matrix component and the polyelectrolyte;
   a step of obtaining a cell-containing solution containing the precipitate and a gelling promoter;
   a step of forming a gel by injecting the cell-containing solution into a droplet of a reaction liquid containing a plurality of gelling agents; and
   a culture step of culturing the cells in the gel, wherein
   the polyelectrolyte includes heparin,
   a concentration of the polyelectrolyte is greater than 0 mg/mL and 1.5 mg/mL or less relative to a total amount of the cell-containing solution,
   the extracellular matrix component includes collagen,
   a concentration of the extracellular matrix component is greater than 0 mg/mL and 1.5 mg/mL or less relative to a total amount of the cell-containing solution,
   the plurality of gelling agents include fibrinogen and sodium alginate, and
   the gelling promoter includes thrombin and calcium ions.

### [Advantageous Effects of Invention]

According to the present invention, a method for producing a cell structure that can suppress variations in angiogenic ability can be provided.

### [Brief Description of Drawings]

Fig. 1 includes images showing the results of evaluating angiogenesis in a cell structure produced by the method of Test Example 1.
Fig. 2 is an image showing the result of evaluating angiogenesis in a cell structure produced by the method of Test Example 2.
Fig. 3 is an image showing the result of evaluating angiogenesis in a cell structure produced by the method of Test Example 3.
Fig. 4 includes images showing the results of evaluating angiogenesis in a cell structure produced by the method of Test Example 4.
Fig. 5 is an image showing an observation result of a cell structure produced by the method of Test Example 5.
Fig. 6 is an image showing an observation result of a cell structure produced by the method of Test Example 6.
Fig. 7 includes images showing observation results of cell structures produced by the method of Test Example 7.
Fig. 8 is a schematic diagram illustrating an embodiment of a method for producing a cell structure, in which (a) shows a gel containing cells that are precursors of the cell structure, and (b) shows a cell structure obtained by culturing the gel containing the cells in a culture medium.

### [Description of Embodiments]

An embodiment of the present invention will be described in detail below. It should be noted that the present invention is not limited to the following embodiment.

### [Method for Producing Cell Structure]

A method according to the present embodiment is a method for producing a cell structure having a vascular structure, the method including: a gelation step of gelling a cell-containing solution containing cells including vascular endothelial cells to form a gel containing the cells; and a culture step of culturing the cells in the gel, wherein the gel containing the cells is formed by bringing a plurality of gelling agents into contact with a gelling promoter. The method according to the present embodiment may further include a preparation step of preparing a cell-containing solution prior to the gelation step.

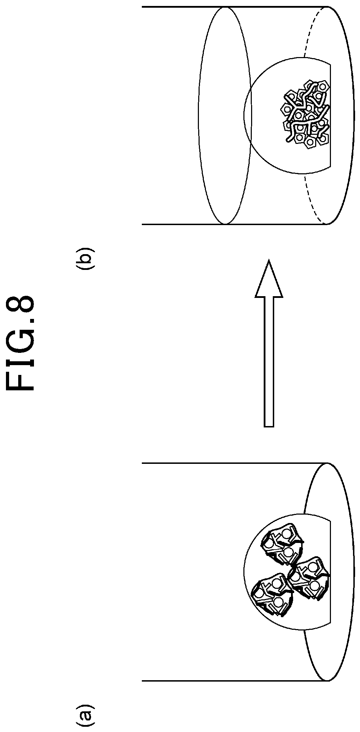

The term "cell structure" as used herein refers to an aggregate of cells (a cluster of cells) in which a plurality of cells are three-dimensionally arranged, which is artificially produced by cell culture. The cell structure may contain one or more types of cells. Fig. 8 is a schematic diagram illustrating an embodiment of a method for producing a cell structure including a gelation step and a culture step. In Fig. 8, (a) shows a gel containing cells after the gelation step and before the culture step. In Fig. 8, (b) shows a cell structure formed by culturing the gel containing the cells shown in (a) in a culture medium. As shown in Fig. 8(b), the cell structure includes a cell aggregate composed of a plurality of cells, and the cell aggregate is embedded in the gel. As shown in Fig. 8(b), a part of the cell aggregate in the cell structure is in contact with the bottom of the culture vessel, and the gel is formed so as to cover the cell aggregate.

The term "cell structure having a vascular structure" as used herein refers to a cell structure having a tubular structure formed of vascular endothelial cells. Blood vessels can be examined by, for example, immunohistochemical staining, microscopic observation using vascular endothelial cells that express fluorescent proteins, and the like. Having a vascular structure means that, for example, when observing the cell structure with a microscope from above, blood vessels are formed in such a manner that a network structure having multiple branch points can be observed.

The shape of the cell structure is not particularly limited, and examples thereof include a sphere, a substantially sphere, an ellipsoid, a substantially ellipsoid, a hemisphere, a substantially hemisphere, a semicircle, a substantially semicircle, a rectangular parallelepiped, and a substantially rectangular parallelepiped. Here, biological tissue includes sweat glands, lymphatic vessels, sebaceous glands, and the like, and has a more complex structure than cell structures. Therefore, cell structures can be easily distinguished from biological tissues.

The term "gelling agent" as used herein refers to a material that forms a gel (hydrogel) by reaction with a gelling promoter, and is in a state prior to gel formation. The term "gelling promoter" as used herein refers to a component that causes gelation of a liquid containing a gelling agent by reacting with a gelling agent. The gelling agent and the gelling promoter are not specifically limited as long as they are materials capable of forming a gel containing cells inside. The gelling agent and the gelling promoter may each be a chemical substance or an enzyme.

Examples of combinations of the gelling agent and the gelling promoter include a combination of fibrinogen and thrombin, a combination of sodium alginate and calcium ions, a combination of collagen and sodium chloride, and a combination of pectin and calcium sulfate.

A plurality of gelling agents at least include a first gelling agent and a second gelling agent different from the first gelling agent. The first gelling agent and the second gelling agent may be a combination of gelling agents having different solidification rates and different elastic moduli after gelation when used alone at a predetermined concentration. For example, when using two gelling agents having different solidification rates, it may be possible to compensate for the insufficient solidification rate of a single gelling agent with appropriate parameters for elastic modulus. Alternatively, when using gelling agents having different elastic moduli, it may be possible to compensate for the insufficient elastic modulus of a single gelling agent with appropriate parameters for solidification rate. The gelling agents used are two or more types, and may be, for example, five or less, four or less, or three or less types, and two types are preferred. Containing a plurality of gelling agents can increase the droplet amount during gel formation. This suppresses cell diffusion and aggregation in the gel, stabilizing the angiogenic ability in the method for producing a cell structure. Containing a plurality of gelling agents improves the shape retention capability of the produced cell structure compared with a case where only a single gelling agent is contained.

The ratio of the mass of the second gelling agent to the mass of the first gelling agent can be adjusted as appropriate according to the type of the gelling agent. The ratio of the mass of the second gelling agent to the mass of the first gelling agent may be, for example, 1 or greater, and preferably 2 or greater, and may be 10 or less, and preferably 5 or less.

The gelling promoter may at least include a first gelling promoter that promotes the gelation reaction by reacting with the first gelling agent, and a second gelling promoter that promotes the gelation reaction by reacting with the second gelling agent. The ratio of the mass of the second gelling promoter to the mass of the first gelling promoter can be adjusted as appropriate according to the type of the gelling promoter. The ratio of the mass of the second gelling promoter to the mass of the first gelling promoter may be, for example, 1 or greater, and preferably 2 or greater, and may be 10 or less, and preferably 5 or less.

From the viewpoint of cytotoxicity, stability, and the like, the plurality of gelling agents may include fibrinogen and sodium alginate. In this case, the gelling promoter may include thrombin and calcium ions.

An example method for producing a cell structure having a vascular structure, including a preparation step, a gelation step and a culture step, will be described below.

### <Preparation Step>

In the preparation step, a cell-containing solution containing cells including vascular endothelial cells is prepared.

The cell-containing solution may contain two or more gelling promoters or two or more gelling agents. When two or more gelling promoters are contained, the cell-containing solution may contain, for example, thrombin and calcium ions. When two or more gelling agents are contained, the cell-containing solution may contain, for example, fibrinogen and sodium alginate. The cell-containing solution may contain a first gelling agent and a second gelling promoter (a second gelling promoter that does not react with the first gelling agent to form a gel). In this case, the cell-containing solution may contain thrombin and sodium alginate, or fibrinogen and calcium ions.

The amount of the gelling agent and/or the gelling promoter used can be appropriately set depending on the types of the gelling agent and the gelling promoter, and the like. The total content of the gelling agent and/or the gelling promoter in the cell-containing solution may be 0.1 mg/mL or greater, preferably 0.5 mg/mL or greater, more preferably 1 mg/mL or greater, even more preferably 2 mg/mL or greater, still even more preferably 3 mg/mL or greater, still even more preferably 4 mg/mL or greater, and still even more preferably 5 mg/mL or greater, and may be 100 mg/mL or less, preferably 90 mg/mL or less, more preferably 80 mg/mL or less, even more preferably 70 mg/mL or less, still even more preferably 60 mg/mL or less, and still even more preferably 50 mg/mL or less, relative to the total amount of the cell-containing solution.

When the cell-containing solution contains fibrinogen, the fibrinogen concentration may be 0.1 mg/ml or greater, preferably 0.5 mg/ml or greater, more preferably 1.0 mg/ml or greater, even more preferably 1.5 mg/ml or greater, still even more preferably 2.0 mg/ml or greater, still even more preferably 2.5 mg/ml or greater, still even more preferably 3.0 mg/ml or greater, still even more preferably 3.5 mg/ml or greater, still even more preferably 4.0 mg/ml or greater, and still even more preferably 4.5 mg/ml or greater, relative to the total amount of the cell-containing solution. When the cell-containing solution contains fibrinogen, the fibrinogen concentration may be 100 mg/ml or less, preferably 90 mg/ml or less, more preferably 80 mg/ml or less, even more preferably 70 mg/ml or less, still even more preferably 60 mg/ml or less, still even more preferably 50 mg/ml or less, still even more preferably 40 mg/ml or less, still even more preferably 30 mg/ml or less, still even more preferably 20 mg/ml or less, still even more preferably 10 mg/ml or less, still even more preferably 9.0 mg/ml or less, still even more preferably 8.0 mg/ml or less, still even more preferably 7.0 mg/ml or less, still even more preferably 6.0 mg/ml or less, and still even more preferably 5.5 mg/ml or less, relative to the total amount of the cell-containing solution. When the cell-containing solution contains fibrinogen, the fibrinogen concentration may be 0.1 mg/ml to 100 mg/ml, preferably 0.5 mg/ml to 50 mg/ml, more preferably 1 mg/ml to 20 mg/ml, even more preferably 2 mg/ml to 10 mg/ml, still even more preferably 3 mg/ml to 8 mg/ml, and still even more preferably 4 mg/ml to 6 mg/ml (e.g., 5 mg/ml), relative to the total amount of the cell-containing solution. When the fibrinogen concentration is within the above range, the three-dimensional structure of the cell structure after tissue preparation becomes more stable.

When the cell-containing solution contains thrombin, the thrombin concentration may be 1 unit/ml or greater, preferably 2 unit/ml or greater, more preferably 4 unit/ml or greater, even more preferably 6 unit/ml or greater, still even more preferably 8 unit/ml or greater, still even more preferably 10 unit/ml or greater, still even more preferably 12 unit/ml or greater, still even more preferably 14 unit/ml or greater, still even more preferably 16 unit/ml or greater, still even more preferably 18 unit/ml or greater, still even more preferably 19 unit/ml or greater, and still even more preferably 19.5 unit/ml or greater, relative to the total amount of the cell-containing solution. When the cell-containing solution contains thrombin, the thrombin concentration may be 1,000 unit/ml or less, preferably 800 unit/ml or less, more preferably 600 unit/ml or less, even more preferably 400 unit/ml or less, still even more preferably 200 unit/ml or less, still even more preferably 100 unit/ml or less, still even more preferably 80 unit/ml or less, still even more preferably 60 unit/ml or less, still even more preferably 40 unit/ml or less, still even more preferably 30 unit/ml or less, and still even more preferably 25 unit/ml or less, relative to the total amount of the cell-containing solution. When the cell-containing solution contains thrombin, the thrombin concentration may be 1 unit/ml to 1,000 unit/ml, preferably 2 unit/ml to 500 unit/ml, more preferably 5 unit/ml to 100 unit/ml, even more preferably 10 unit/ml to 50 unit/ml, and still even more preferably 15 unit/ml to 30 unit/ml (e.g., 20 unit/ml), relative to the total amount of the cell-containing solution. When the thrombin concentration is within the above range, the time from the start of the reaction with fibrinogen to the formation of a gel becomes more stable, and even when a culture medium is added thereafter, a risk of gel damage is low, enabling formation of a stable cell structure.

When the cell-containing solution contains sodium alginate, the sodium alginate content may be 0.01 mass% or greater, preferably 0.02 mass% or greater, more preferably 0.03 mass% or greater, and even more preferably 0.04 mass% or greater, relative to the total amount of the cell-containing solution. When the cell-containing solution contains sodium alginate, the sodium alginate content may be 2.0 mass% or less, preferably 1.5 mass% or less, more preferably 1.0 mass% or less, even more preferably 0.50 mass% or less, still even more preferably 0.10 mass% or less, still even more preferably 0.08 mass% or less, and still even more preferably 0.06 mass% or less, relative to the total amount of the cell-containing solution. When the cell-containing solution contains sodium alginate, the sodium alginate content may be 0.01 mass% to 2 mass%, preferably 0.01 mass% to 1.5 mass%, more preferably 0.02 mass% to 1.0 mass%, even more preferably 0.02 mass% to 0.50 mass%, still even more preferably 0.03 mass% to 0.10 mass%, and still even more preferably 0.03 mass% to 0.08 mass% (e.g., 0.05 mass%), relative to the total amount of the cell-containing solution. When the sodium alginate content is within the above range, the time from the start of the reaction with calcium ions to the formation of a gel becomes more stable, and even when a culture medium is added thereafter, a risk of gel damage is low, enabling formation of a stable cell structure.

When the cell-containing solution contains calcium ions, the calcium ion concentration may be 1.0 mM or greater, preferably 2.0 mM or greater, more preferably 3.0 mM or greater, even more preferably 4.0 mM or greater, still even more preferably 4.5 mM or greater, and still even more preferably 5.0 mM or greater, relative to the total amount of the cell-containing solution. When the cell-containing solution contains calcium ions, the calcium ion concentration may be 200 mM or less, preferably 150 mM or less, more preferably 100 mM or less, even more preferably 80 mM or less, still even more preferably 60 mM or less, still even more preferably 40 mM or less, still even more preferably 20 mM or less, still even more preferably 10 mM or less, still even more preferably 8 mM or less, and still even more preferably 6 mM or less, relative to the total amount of the cell-containing solution. When the cell-containing solution contains calcium ions, the calcium ion concentration may be 1 mM to 200 mM, preferably 1 mM to 150 mM, more preferably 2 mM to 100 mM, even more preferably 2 mM to 50 mM, still even more preferably 5 mM to 20 mM, and still even more preferably 5 mM to 10 mM, relative to the total amount of the cell-containing solution. The cell-containing solution containing calcium ions can be prepared by adding a calcium salt such as calcium chloride to the cell-containing solution. When the calcium ion concentration is within the above range, the time from the start of the reaction with sodium alginate to the formation of a gel becomes more stable, and even when a culture medium is added thereafter, a risk of gel damage is low, enabling formation of a stable cell structure.

### (Cells)

The cells include at least vascular endothelial cells. Vascular endothelial cells refer to flattened cells constituting the surface of the lumen of blood vessels. The vascular endothelial cells in the culture step may be, for example, sinusoidal endothelial cells or human umbilical vein-derived endothelial cells (HUVECs). Sinusoidal endothelial cells are non-parenchymal liver cells (cells other than hepatocytes among the cells constituting the liver) that have a characteristic morphology that differs from other vascular endothelial cells, such as having numerous clusters of small pores in their cytoplasm (cribrosa structure) and lacking a basement membrane. The vascular endothelial cells in the culture step may be primary cells (primary vascular endothelial cells) collected from an animal liver (e.g., human liver), cells obtained by culturing primary cells, a cultured cell line established from primary cells, or cells artificially differentiated from stem cells. The cultured cell line may be established by gene introduction, specifically by introducing immortalization-inducing genes such as hTERT or SV40LT. Examples of the primary vascular endothelial cells include primary sinusoidal endothelial cells such as product number 5000 from ScienCell. When a cultured cell line immortalized by gene introduction into primary sinusoidal endothelial cells is used, the effects of growth factors involved in angiogenesis on formation and maintenance of blood vessels are expected to be further improved. Examples of the cultured cell line include a cultured cell line with product number T0056 from Applied Biological Materials. Examples of stem cells to be differentiated include embryonic stem cells (ES cells) and induced pluripotent stem cells (iPS cells). The vascular endothelial cells in the culture step may be non-cancerous cells.

The cells may further include epithelial cells. Epithelial cells are cells constituting epithelial tissues present in the skin, digestive organs, blood vessels, and the like. Examples of the epithelial cells include hepatocytes, epidermal keratinocytes, renal epithelial cells, vascular epithelial cells, alveolar epithelial cells, intestinal epithelial cells, retinal epithelial cells, neural epithelial cells, gingival epithelial cells, bile duct epithelial cells and thymic epithelial cells.

The cells preferably include hepatocytes. Hepatocytes, or hepatic parenchymal cells, are cells having functions such as bile secretion and plasma protein secretion. The hepatocytes constituting the cell structure may be primary hepatocytes collected from an animal liver, cells obtained by culturing primary hepatocytes, a cultured cell line established from primary hepatocytes, or hepatoblasts artificially differentiated from stem cells. Examples of the primary hepatocytes include frozen primary hepatocytes and primary hepatocytes, such as PXB Cells (registered trademark) and HepaSH (registered trademark), collected from a human hepatocyte chimeric animal in which primary human hepatocytes have been transplanted into the liver of an animal such as a mouse. Examples of cultured cell line include cell lines derived from inactivated hepatoma cells, such as HepG2. Examples of stem cells differentiated into hepatoblasts include embryonic stem cells (ES cells), induced pluripotent stem cells (iPS cells) and mesenchymal stem cells. The hepatocytes are preferably non-cancerous cells such as primary hepatocytes and hepatoblasts, and more preferably PXB cells due to their ease of handling. The hepatocytes used may preferably be functionally normal and mature hepatocytes since the cell structure can exhibit a high liver function similar to that of the original living body, and specifically, primary hepatocytes, HepaRG (registered trademark) and hepatocytes highly differentiated from stem cells can be used. In general, when such functionally mature hepatocytes are used and co-cultured in a cell structure containing vascular endothelial cells, it tends to be difficult for the vascular endothelial cells to form and maintain a vascular network in the tissue, but according to a method of an aspect of the present invention, it is possible to form and maintain a vascular network.

The cells may include one or more types of epithelial cells. For example, the cells may include a plurality of hepatocytes having different genotypes of proteins involved in liver function. All hepatocytes included in the cells may have the same genotype of proteins involved in liver function. Examples of the proteins involved in liver function include drug metabolizing enzymes.

The cells may further include hepatic stellate cells. Hepatic stellate cells are non-parenchymal liver cells (cells other than hepatocytes among the cells constituting the liver) that have functions such as storing vitamin A and are present in the space of Disse, the region between hepatocytes and the sinusoids in the liver. The hepatic stellate cells may be primary hepatic stellate cells collected from the liver, or may be cells obtained by culturing primary hepatic stellate cells. The hepatic stellate cells may be a cell line established from primary cells, or hepatic stellate cells artificially differentiated from stem cells. Examples of the hepatic stellate cells include hepatic stellate cell lines such as LX2.

The cells may further include other cells than epithelial cells, vascular endothelial cells and hepatic stellate cells. Such other cells may be, for example, mature somatic cells, or may be undifferentiated cells such as stem cells. Specific examples of somatic cells include nerve cells, dendritic cells, immune cells, lymphatic endothelial cells, fibroblasts, myocardial cells, islet cells, smooth muscle cells, bone cells and spleen cells. Examples of stem cells include ES cells, iPS cells and mesenchymal stem cells. The other cells may be normal cells, or cells whose cellular function is enhanced or suppressed, such as cancer cells. "Cancer cells" are cells that are derived from somatic cells and have acquired infinite proliferation potential.

The origin of the cells is not specifically limited, and may be, for example, cells derived from mammals such as humans, monkeys, dogs, cats, rabbits, pigs, cows, mice and rats.

The total number of cells (X0) is not specifically limited, and may be determined as appropriate taking into consideration factors such as the thickness and shape of the cell structure to be produced, and the size of the cell culture vessel to be used for its production. The total number of cells (X0) may be 1×10³ cells or more, preferably 1×10⁴ cells or more, more preferably 1×10⁵ cells or more, and even more preferably 1×10⁶ cells or more. The total number of cells (X0) may be 1×10⁹ cells or less, preferably 1×10⁷ cells or less, and more preferably 1×10⁵ cells or less.

The ratio (X1/X0 × 100) of the number of vascular endothelial cells (X1) to the total number of cells (X0) may be 5% or greater, preferably 10% or greater, more preferably 15% or greater, and even more preferably 20% or greater, and may be 50% or less, preferably 45% or less, more preferably 40% or less, even more preferably 35% or less, and still even more preferably 30% or less, from the viewpoint of being more suitable as a cell structure having blood vessels. Preferably, the ratio of the vascular endothelial cells to the total number of cells is 20% or greater and 30% or less from the viewpoint of being more suitable as a cell structure having blood vessels.

The ratio (X2/X0 × 100) of the number of epithelial cells (X2) to the total number of cells (X0) may be 5% or greater, preferably 10% or greater, more preferably 15% or greater, even more preferably 20% or greater, still even more preferably 25% or greater, still even more preferably 30% or greater, still even more preferably 35% or greater, still even more preferably 40% or greater, still even more preferably 45% or greater, still even more preferably 50% or greater, still even more preferably 55% or greater, still even more preferably 60% or greater, and still even more preferably 65% or greater, and may be 95% or less, preferably 90% or less, more preferably 80% or less, even more preferably 75% or less, and still even more preferably 70% or less. Preferably, the ratio of the epithelial cells to the total number of cells is 60% or greater and 80% or less from the viewpoint of being more suitable as a cell structure having epithelial cells. When the epithelial cells are hepatocytes, the ratio of the hepatocytes is preferably 50% or greater, which is a condition similar to that of a living body.

The ratio (X3/X0 × 100) of the number of hepatic stellate cells (X3) to the total number of cells (X0) may be 1% or greater, preferably 3% or greater, more preferably 5% or greater, even more preferably 8% or greater, and still even more preferably 10% or greater, and may be 30% or less, preferably 25% or less, more preferably 20% or less, even more preferably 15% or less, and still even more preferably 10% or less, from the viewpoint of being more suitable as a cell structure having hepatic stellate cells. Preferably, the ratio of the hepatic stellate cells to the total number of cells is 5% or greater and 15% or less from the viewpoint of being more suitable as a cell structure having hepatic stellate cells.

### (Extracellular Matrix Component)

The cell-containing solution may further contain an extracellular matrix component. When the cell-containing solution contains an extracellular matrix component, cell accumulation is promoted. This promotes adhesion between vascular endothelial cells, making it easier to further suppress variation in angiogenic ability.

The term "extracellular matrix component" as used herein refers to an aggregate of extracellular matrix molecules formed of a plurality of extracellular matrix molecules. An extracellular matrix molecule refers to a substance that exists outside the cells in an organism. As the extracellular matrix, any suitable substance can be used as long as it does not adversely affect cell growth and cell aggregate formation. Specific examples of the extracellular matrix molecules include, but are not limited to, collagen, elastin, proteoglycans, fibronectin, hyaluronic acid, laminin, vitronectin, tenascin, entactin, fibrillin and cadherin. The extracellular matrix components may be used singly or in combination.

The extracellular matrix may be a modification or variant of the above extracellular matrix, or may be a polypeptide such as a chemically synthesized peptide, as long as it does not adversely affect cell growth and cell aggregate formation. The extracellular matrix may have repeats of the sequence represented by Gly-X-Y, which is characteristic of collagen. Gly represents a glycine residue, and X and Y each independently represent a suitable amino acid residue. The plurality of Gly-X-Y sequences may be the same or different. By having repeats of the sequence represented by Gly-X-Y, restrictions on the arrangement of the molecular chain are reduced, and therefore, for example, the function as a scaffold material for cell culture is further improved. In an extracellular matrix having repeats of the sequence represented by Gly-X-Y, the proportion of the sequence represented by Gly-X-Y in the entire amino acid sequence may be 80% or greater, and preferably 95% or greater. The extracellular matrix may also be a polypeptide having an RGD sequence. The RGD sequence refers to a sequence represented by Arg-Gly-Asp (arginine residue-glycine residue-aspartic acid residue). Having the RGD sequence further promotes cell adhesion, making the extracellular matrix more suitable as a scaffold material for cell culture, for example. Examples of the extracellular matrices having a sequence represented by Gly-X-Y and an RGD sequence include collagen, fibronectin, vitronectin, laminin and cadherin.

Examples of the collagen include fibrillar collagen and non-fibrillar collagen. Fibrillar collagen refers to collagen that is the main component of collagen fibers, and specific examples include type I collagen, type II collagen and type III collagen. Examples of non-fibrillar collagen include type IV collagen.

Examples of the proteoglycan include, but are not limited to, chondroitin sulfate proteoglycan, heparan sulfate proteoglycan, keratan sulfate proteoglycan and dermatan sulfate proteoglycan.

The shape of the extracellular matrix component may be, for example, fibrous. Fibrous refers to a shape composed of a thread-like extracellular matrix component, or a shape composed of crosslinked molecules of a thread-like extracellular matrix component. At least a portion of the extracellular matrix components may be fibrous. The shape of the extracellular matrix component is the shape of a single clump of the extracellular matrix component (an extracellular matrix component aggregate) observed under a microscope, and the extracellular matrix component preferably has a mean diameter and/or mean length as described below. A fibrous extracellular matrix component includes thin threads (fibrils) formed by the assembly of a plurality of thread-like extracellular matrix molecules, threads formed by the further assembly of fibrils, and defibrillated versions of these threads. When an extracellular matrix component having a fibrous shape is contained, the RGD sequence is preserved without being destroyed in the fibrous extracellular matrix component, and therefore it can function even more effectively as a scaffold for cell adhesion.

The extracellular matrix component may contain at least one selected from the group consisting of collagen, laminin and fibronectin, and preferably contains collagen. The collagen is preferably fibrillar collagen, and more preferably type I collagen. The fibrillar collagen may be a commercially available collagen, and specific examples thereof include type I collagen derived from pig skin manufactured by NH Foods Ltd.

The extracellular matrix component may be an extracellular matrix component derived from an animal. Examples of animal species from which the extracellular matrix component may be derived include, but are not limited to, humans, pigs and cows. Extracellular matrix components may be derived from one type of animal, or may be a combination of components derived from a plurality of types of animals.

The content of the extracellular matrix component can be determined as appropriate depending on the shape, thickness, and the like of the target cell structure. The content of the extracellular matrix component may be, for example, 0.005 mg/mL or greater, preferably 0.01 mg/mL or greater, more preferably 0.025 mg/mL or greater, even more preferably 0.05 mg/mL or greater, still even more preferably 0.10 mg/mL or greater, still even more preferably 0.15 mg/mL or greater, still even more preferably 0.20 mg/mL or greater, still even more preferably 0.25 mg/mL or greater, still even more preferably 0.30 mg/mL or greater, still even more preferably 0.35 mg/mL or greater, still even more preferably 0.40 mg/mL or greater, and still even more preferably 0.45 mg/mL or greater, and may be 1.5 mg/mL or less, preferably 1.25 mg/mL or less, more preferably 1.0 mg/mL or less, even more preferably 0.8 mg/mL or less, and still even more preferably 0.6 mg/mL or less, relative to the total amount of the cell-containing solution. When the content of the extracellular matrix component is within the above range, adhesion between cells is more easily promoted, the three-dimensional structure becomes more stable, and formation of a vascular network becomes more stable.

### (Polyelectrolyte)

The cell-containing solution may further contain a polyelectrolyte. A polyelectrolyte is a polymer compound having electrolyte properties. When the cell-containing solution contains a polyelectrolyte, cell accumulation is promoted. This promotes adhesion between vascular endothelial cells, making it easier to further suppress variation in angiogenic ability.

Examples of the polyelectrolyte include, but are not limited to, glycosaminoglycans such as heparin, chondroitin sulfate (e.g., chondroitin 4-sulfate and chondroitin 6-sulfate), heparan sulfate, dermatan sulfate, keratan sulfate and hyaluronic acid; dextran sulfate, rhamnan sulfate, fucoidan, carrageenan, polystyrene sulfonic acid, polyacrylamide-2-methylpropanesulfonic acid, polyacrylic acid, and derivatives thereof. These polyelectrolytes may be used singly or in combination of two or more.

The polyelectrolyte is preferably a glycosaminoglycan, more preferably contains at least one selected from the group consisting of heparin, dextran sulfate, chondroitin sulfate and dermatan sulfate, and even more preferably contains heparin.

The concentration of the polyelectrolyte in the cell-containing solution is not particularly limited as long as it does not adversely affect the cell growth and formation of a cell structure. The concentration of the polyelectrolyte may be, for example, greater than 0 mg/mL and 1.5 mg/mL or less relative to the total amount of the cell-containing solution. The concentration of the polyelectrolyte may be 0.005 mg/mL or greater, preferably 0.01 mg/mL or greater, more preferably 0.02 mg/mL or greater, even more preferably 0.03 mg/mL or greater, and still even more preferably 0.04 mg/mL or greater, and may be 1.5 mg/mL or less, preferably 1.0 mg/mL or less, more preferably 0.1 mg/mL or less, even more preferably 0.08 mg/mL or less, and still even more preferably 0.06 mg/mL or less, relative to the total amount of the cell-containing solution. The concentration of the polyelectrolyte may be, for example, 0.025 mg/mL, 0.05 mg/mL, 0.075 mg/mL or 0.1 mg/mL, relative to the total amount of the cell-containing solution. When the concentration of the polyelectrolyte is within the above range, adhesion between cells is more easily promoted while suppressing excessive aggregation between cells, the three-dimensional structure becomes stable, and formation of a vascular network becomes stable.

The ratio (C2/C1) of a mass C2 of the polyelectrolyte to a mass C1 of the extracellular matrix component may be 1/100 to 100/1, preferably 1/10 to 10/1, more preferably 1/5 to 5/1, even more preferably 1/2 to 2/1, and still even more preferably 1/1.5 to 1.5/1. When the ratio (C2/C1) of the mass C2 of the polyelectrolyte to the mass C1 of the extracellular matrix component is within the above range, adhesion between vascular endothelial cells is more easily promoted, and variation in angiogenic ability is more easily suppressed.

### (Aqueous Medium)

The cell-containing solution contains an aqueous medium. The term "aqueous medium" refers to a liquid containing water as an essential component. The aqueous medium may be, for example, an aqueous medium containing a cationic substance. The aqueous medium containing a cationic substance may be, for example, a cationic buffer solution such as a tris-hydrochloric acid buffer, tris-maleic acid buffer, Bis-Tris buffer, or HEPES (4-(2-hydroxyethyl)-1-piperazineethanesulfonic acid), or may be a medium containing water and a cationic compound such as ethanolamine, diethanolamine, triethanolamine, polyvinylamine, polyallylamine, polylysine, polyhistidine, or polyarginine as the cationic substance. A culture medium can also be used as the aqueous medium. Examples of the culture medium include liquid media such as Dulbecco's Modified Eagle's Medium (DMEM) and hepatocyte culture media (HCM). The liquid medium may be a mixed medium in which two types of culture media are mixed.

The concentration and pH of the cationic substance in an aqueous medium containing the cationic substance (e.g., tris in a tris-hydrochloric acid buffer solution) are not specifically limited as long as they do not adversely affect the cell growth and formation of a cell structure. For example, the concentration of the cationic substance may be 10 mM to 100 mM, preferably 20 mM to 80 mM, more preferably 40 mM to 70 mM, even more preferably 40 mM to 60 mM, and still even more preferably 50 mM, relative to the total amount of the aqueous medium containing the cationic substance. The pH of the aqueous medium (e.g., cationic buffer) may be 6.0 to 8.0, preferably 6.8 to 7.8, and more preferably 7.2 to 7.6.

### (Method for Producing Cell-Containing Solution)

A cell-containing solution can be obtained by a method including mixing cells, a plurality of gelling agents, an aqueous medium, and, if necessary, an extracellular matrix component and a polyelectrolyte. The mixing order is not specifically limited. The method for producing a cell-containing solution may include, if necessary, centrifuging the mixed solution. Specific examples of the method for producing a cell-containing solution include a method including mixing cells, an extracellular matrix component and a polyelectrolyte to obtain a mixed solution, centrifuging the mixed solution to obtain a precipitate containing the cells, the extracellular matrix component and the polyelectrolyte, and mixing a plurality of gelling agents with the precipitate to obtain a cell-containing solution.

### <Gelation Step>

In the gelation step, a cell-containing solution containing cells is gelled to form a gel containing the cells. The gelation may be performed by bringing the cell-containing solution into contact with a reaction liquid containing an aqueous medium, a gelling agent and/or a gelling promoter. The type of the gelling agent and/or the gelling promoter in the reaction liquid is determined depending on the type of the gelling agent and/or the gelling promoter in the cell-containing solution. The aqueous medium may be as described above.

The amount of the gelling agent and/or the gelling promoter used in the gelation of the cell-containing solution can be appropriately set depending on the types of the gelling agent and the gelling promoter, and the like. The total content of the gelling agent and/or the gelling promoter in the reaction liquid may be 0.1 mg/mL or greater, preferably 0.5 mg/mL or greater, more preferably 1 mg/mL or greater, even more preferably 2 mg/mL or greater, still even more preferably 3 mg/mL or greater, still even more preferably 4 mg/mL or greater, and still even more preferably 5 mg/mL or greater, and may be 100 mg/mL or less, preferably 90 mg/mL or less, more preferably 80 mg/mL or less, even more preferably 70 mg/mL or less, still even more preferably 60 mg/mL or less, and still even more preferably 50 mg/mL or less, relative to the total amount of the reaction liquid.

When the reaction liquid contains fibrinogen, the fibrinogen concentration may be 0.1 mg/ml or greater, preferably 0.5 mg/ml or greater, more preferably 1.0 mg/ml or greater, even more preferably 1.5 mg/ml or greater, still even more preferably 2.0 mg/ml or greater, still even more preferably 2.5 mg/ml or greater, still even more preferably 3.0 mg/ml or greater, still even more preferably 3.5 mg/ml or greater, still even more preferably 4.0 mg/ml or greater, and still even more preferably 4.5 mg/ml or greater, relative to the total amount of the reaction liquid. When the reaction liquid contains fibrinogen, the fibrinogen concentration may be 100 mg/ml or less, preferably 90 mg/ml or less, more preferably 80 mg/ml or less, even more preferably 70 mg/ml or less, still even more preferably 60 mg/ml or less, still even more preferably 50 mg/ml or less, still even more preferably 40 mg/ml or less, still even more preferably 30 mg/ml or less, still even more preferably 20 mg/ml or less, still even more preferably 10 mg/ml or less, still even more preferably 9.0 mg/ml or less, still even more preferably 8.0 mg/ml or less, still even more preferably 7.0 mg/ml or less, still even more preferably 6.0 mg/ml or less, and still even more preferably 5.5 mg/ml or less, relative to the total amount of the reaction liquid. When the reaction liquid contains fibrinogen, the fibrinogen concentration may be 0.1 mg/ml to 100 mg/ml, preferably 0.5 mg/ml to 50 mg/ml, more preferably 1 mg/ml to 20 mg/ml, even more preferably 2 mg/ml to 10 mg/ml, still even more preferably 3 mg/ml to 8 mg/ml, and still even more preferably 4 mg/ml to 6 mg/ml (e.g., 5 mg/ml), relative to the total amount of the reaction liquid. When the fibrinogen concentration is within the above range, the three-dimensional structure of the cell structure after tissue preparation becomes more stable.

When the reaction liquid contains thrombin, the thrombin concentration may be 1 unit/ml or greater, preferably 2 unit/ml or greater, more preferably 4 unit/ml or greater, even more preferably 6 unit/ml or greater, still even more preferably 8 unit/ml or greater, still even more preferably 10 unit/ml or greater, still even more preferably 12 unit/ml or greater, still even more preferably 14 unit/ml or greater, still even more preferably 16 unit/ml or greater, still even more preferably 18 unit/ml or greater, still even more preferably 19 unit/ml or greater, and still even more preferably 19.5 unit/ml or greater, relative to the total amount of the reaction liquid. When the reaction liquid contains thrombin, the thrombin concentration may be 1,000 unit/ml or less, preferably 800 unit/ml or less, more preferably 600 unit/ml or less, even more preferably 400 unit/ml or less, still even more preferably 200 unit/ml or less, still even more preferably 100 unit/ml or less, still even more preferably 80 unit/ml or less, still even more preferably 60 unit/ml or less, still even more preferably 40 unit/ml or less, still even more preferably 30 unit/ml or less, and still even more preferably 25 unit/ml or less, relative to the total amount of the reaction liquid. When the reaction liquid contains thrombin, the thrombin concentration may be 1 unit/ml to 1,000 unit/ml, preferably 2 unit/ml to 500 unit/ml, more preferably 5 unit/ml to 100 unit/ml, even more preferably 10 unit/ml to 50 unit/ml, and still even more preferably 15 unit/ml to 30 unit/ml (e.g., 20 unit/ml), relative to the total amount of the reaction liquid. When the thrombin concentration is within the above range, the time from the start of the reaction with fibrinogen to the formation of a gel becomes more stable, and even when a culture medium is added thereafter, a risk of gel damage is low, enabling formation of a stable cell structure.

When the reaction liquid contains sodium alginate, the sodium alginate content may be 0.01 mass% or greater, preferably 0.02 mass% or greater, more preferably 0.03 mass% or greater, and even more preferably 0.04 mass% or greater, relative to the total amount of the reaction liquid. When the reaction liquid contains sodium alginate, the sodium alginate content may be 2.0 mass% or less, preferably 1.5 mass% or less, more preferably 1.0 mass% or less, even more preferably 0.50 mass% or less, still even more preferably 0.10 mass% or less, still even more preferably 0.08 mass% or less, and still even more preferably 0.06 mass% or less, relative to the total amount of the reaction liquid. When the reaction liquid contains sodium alginate, the sodium alginate content may be 0.01 mass% to 2 mass%, preferably 0.01 mass% to 1.5 mass%, more preferably 0.02 mass% to 1.0 mass%, even more preferably 0.02 mass% to 0.50 mass%, still even more preferably 0.03 mass% to 0.10 mass%, and still even more preferably 0.03 mass% to 0.08 mass% (e.g., 0.05 mass%), relative to the total amount of the reaction liquid. When the sodium alginate content is within the above range, the time from the start of the reaction with sodium alginate to the formation of a gel becomes more stable, and even when a culture medium is added thereafter, a risk of gel damage is low, enabling formation of a stable cell structure.

When the reaction liquid contains calcium ions, the calcium ion concentration may be 1.0 mM or greater, preferably 2.0 mM or greater, more preferably 3.0 mM or greater, even more preferably 4.0 mM or greater, still even more preferably 4.5 mM or greater, and still even more preferably 5.0 mM or greater, relative to the total amount of the reaction liquid. When the reaction liquid contains calcium ions, the calcium ion concentration may be 200 mM or less, preferably 150 mM or less, more preferably 100 mM or less, even more preferably 80 mM or less, still even more preferably 60 mM or less, still even more preferably 40 mM or less, still even more preferably 20 mM or less, still even more preferably 10 mM or less, still even more preferably 8 mM or less, and still even more preferably 6 mM or less, relative to the total amount of the reaction liquid. When the reaction liquid contains calcium ions, the calcium ion concentration may be 1 mM to 200 mM, preferably 1 mM to 150 mM, more preferably 2 mM to 100 mM, even more preferably 2 mM to 50 mM, still even more preferably 5 mM to 20 mM, and still even more preferably 5 mM to 10 mM, relative to the total amount of the reaction liquid. The reaction liquid containing calcium ions can be prepared by adding a calcium salt such as calcium chloride to the reaction liquid. When the calcium ion concentration is within the above range, the time from the start of the reaction with sodium alginate to the formation of a gel becomes more stable, and even when a culture medium is added thereafter, a risk of gel damage is low, enabling formation of a stable cell structure.

When the gelation is performed by bringing the cell-containing solution into contact with the reaction liquid, the gelation step may be performed by injecting one of the cell-containing solution and the reaction liquid (either the cell-containing solution or the reaction liquid) (injection liquid) into a droplet of the other liquid, and it is preferred that the cell-containing solution is injected into a droplet of the reaction liquid since this facilitates formation of a gel encapsulating cells. The injection method is not specifically limited, and a conventional injection method can be used.

The droplet amount of one of the liquids (either the cell-containing solution or the reaction liquid) may be 2 µL or greater, preferably 5 µL or greater, more preferably 8 µL or greater, and even more preferably 10 µL or greater, and may be 30 µL or less, preferably 25 µL or less, more preferably 20 µL or less, even more preferably 15 µL or less, and still even more preferably 10 µL or less, from the viewpoint of ease of handling and further improving the effect of suppressing variation in angiogenic ability. The volume ratio (droplet amount (µL) / injection amount (µL)) of the droplet amount of one of the liquids to the injection amount of the other liquid (injection liquid) may be 2 or greater, preferably 3 or greater, and more preferably 4 or greater, and may be 10 or less, preferably 9 or less, more preferably 8 or less, even more preferably 7 or less, and still even more preferably 6 or less, from the viewpoint of further improving the effect of suppressing variation in angiogenic ability.

The gelation step may be performed by injecting one of the cell-containing solution and the reaction liquid into a droplet of the other liquid formed on a substrate. The substrate used can be a conventional culture vessel. The substrate may be a container that has a surface capable of forming a gel and can accommodate a culture medium. The material, shape, and the like of the substrate surface are not specifically limited. Examples of the substrate include a dish, a well insert, and a plate with a bottom shape such as a U-shape or a V-shape. The substrate may be subjected to a surface-treatment. There are no particular restrictions on the surface treatment method or the coating agent that can be used for the surface treatment. From the viewpoint of ease of tissue preparation, the substrate is preferably a culture vessel with a flat bottom.

The gelation step is preferably a step of forming a gel by injecting the cell-containing solution into a droplet of the reaction liquid since this facilitates formation of a gel encapsulating cells or a cell population. The volume ratio of a droplet amount of the reaction liquid to an injection amount of the cell-containing solution is preferably within the above range.

The gelation step may further include, after bringing a plurality of gelling agents into contact with a gelling promoter, performing incubation (gelling culture) for a predetermined period of time. Incubation refers to a process in which a sample is left standing to allow a reaction to proceed. The incubation temperature (gelling culture temperature) may be, for example, 20°C to 40°C, and preferably 30°C to 37°C. The incubation time (gelling culture time) may be, 10 minutes or more, preferably 20 minutes or more, and more preferably 30 minutes or more, and may be 60 minutes or less, preferably 50 minutes or less, and more preferably 40 minutes or less. In conventional methods, the angiogenic ability of the produced cell structure may vary depending on the incubation time after gelation. On the other hand, according to the method of the present embodiment, variations in the angiogenic ability of the cell structure due to the incubation time are suppressed.

### <Culture Step>

In the culture step, cells are cultured in the gel. The culture step can be performed after a culture medium is added to the culture substrate on which a gel containing the cells is disposed. The term "culturing cells" as used herein means maintaining cells in a culture medium under conditions that prevent the cells from dying or declining. Culturing cells may or may not involve proliferation of the cells.

A suitable culture medium can be selected depending on the type of cells to be cultured. Examples of the culture medium include Eagle's MEM medium, DMEM, Modified Eagle medium (MEM), Minimum Essential medium, RPMI, GlutaMax medium, and hepatocyte medium (for example, HCM medium (manufactured by Lonza)). The medium may be a medium supplemented with serum or a serum-free medium. Further, the medium may be a mixed medium in which two types of culture media are mixed. In addition, additives may be diluted to a predetermined concentration and added to the culture medium, if necessary. Examples of the additives include individual growth factors such as VEGF-A and VEGF-A, a cocktail of such additives, and an extract collected from a biological sample, such as Matrigel (registered trademark).

As the cell culture conditions in the culture step, suitable culture conditions can be set depending on the type of cells. For example, the culture temperature may be 20°C to 40°C, and preferably 30°C to 37°C. The pH of the culture medium may be 6 to 8, and preferably 7.2 to 7.4. The culture time may be 1 day to 2 weeks, and preferably 1 week to 2 weeks.

In the cell structure produced by the method according to the present embodiment, variations in angiogenic ability are reduced. Further, in the method according to the present embodiment, evaporation of liquid components occurring during the gelation and gel damage when adding a culture medium in the culture step are suppressed. In addition, since the method according to the present embodiment enables manual preparation of cell structures without requiring the use of a special device for tissue preparation, it can easily produce cell structures with reduced variations in angiogenic ability.

### [Cell Structure]

The cell structure according to the present embodiment contains cells including vascular endothelial cells, and a plurality of gels encapsulating cells. The gel may be formed by the reaction between the gelling agent and the gelling promoter described above, and may include, for example, a fibrin gel and an alginate gel. The cell structure according to the present embodiment may contain an extracellular matrix component, and may further contain a polyelectrolyte. The cell structure according to the present embodiment may be produced by, for example, the method described above.

When the cell structure according to the present embodiment contains an extracellular matrix component, the content of the extracellular matrix component in the cell structure may be 0.01% by mass or greater, preferably 0.05% by mass or greater, more preferably 0.1% by mass or greater, even more preferably 0.5% by mass or greater, still even more preferably 1% by mass or greater, still even more preferably 2% by mass or greater, still even more preferably 3% by mass or greater, still even more preferably 4% by mass or greater, still even more preferably 5% by mass or greater, still even more preferably 6% by mass or greater, still even more preferably 7% by mass or greater, still even more preferably 8% by mass or greater, still even more preferably 9% by mass or greater, still even more preferably 10% by mass or greater, still even more preferably 15% by mass or greater, still even more preferably 20% by mass or greater, still even more preferably 25% by mass or greater, and still even more preferably 30% by mass or greater, and may be 90% by mass or less, preferably 80% by mass or less, more preferably 70% by mass or less, even more preferably 60% by mass or less, still even more preferably 50% by mass or less, still even more preferably 30% by mass or less, still even more preferably 20% by mass or less, and still even more preferably 15% by mass or less, relative to the dry weight of the cell structure. The content of the extracellular matrix component in the cell structure may be 0.01 to 90% by mass, preferably 10 to 90% by mass, more preferably 10 to 80% by mass, even more preferably 10 to 70% by mass, still even more preferably 10 to 60% by mass, still even more preferably 10 to 50% by mass, still even more preferably 10 to 40% by mass, still even more preferably 10 to 30% by mass, and still even more preferably 20 to 30% by mass, relative to the dry weight of the cell structure.

When the cell structure according to the present embodiment is observed with a microscope from the above, the maximum length in a microscopic image may be 1,000 µm or greater, and preferably 2,000 µm or greater, and may be 4,000 µm or less, and preferably 3,000 µm or less. Preferably, the maximum length is 2,000 µm to 3,000 µm from the viewpoint of maintaining the three-dimensional shape retention capability of the cell structure.

### Examples

The present invention will be further described in detail below by using examples. However, the present invention is not limited to these examples.

The following cells were used.
Hepatocytes (PXB Cells (registered trademark), manufactured by PhoenixBio Co., Ltd.)
Hepatic stellate cells (LX2, manufactured by Sigma-Aldrich Inc.)
Vascular endothelial cells (GFP-HUVEC, manufactured by ANGIO-PROTEOMIE)

### <Test Example 1>

LX2 and GFP-HUVEC were awakened and precultured according to the manufacturer's protocol. LX2 and GFP-HUVEC were detached using 0.05% Trypsin/EDTA. PXB Cells were detached using 0.25% Trypsin/EDTA. After the detachment, LX2 and GFP-HUVEC were suspended in DMEM medium containing 10% FBS, and PXB cells were suspended in HCGM medium. A portion of the suspension was mixed with trypan blue, and the cell concentration was measured using a Countess (registered trademark) II FL (Invitrogen). After the measurement, taking into consideration the number of wells to be prepared, a necessary amount of each cell was aliquoted so that the cell seeding amount per well was 19,500 cells for PXB Cells, 7,500 cells for GFP-HUVEC and 3,000 cells for LX2, and the three types of cells were mixed. The resulting mixed solution was centrifuged at 1,200 rpm for 5 minutes, the supernatant was carefully removed, and then the cells were suspended in a heparin collagen solution. The heparin collagen solution was prepared by mixing equal amounts of a 1.0 mg/ml heparin solution and a 0.6 mg/ml collagen solution dissolved in 100 mM Tris buffer. After the suspension, the cell suspension was allowed to stand at room temperature for 3 minutes, and centrifuged at 400 G for 2 minutes. Then, the supernatant was carefully removed, and the cells were suspended in a 20 U/ml thrombin solution dissolved in HCM medium supplemented with ECGS (hereinafter, simply referred to as "HCM medium") at a concentration of 30,000 cells/2 µL. Thus, a cell-containing solution of Test Example 1 containing cells and thrombin as a gelling promoter was prepared.

A 5 mg/ml fibrinogen solution was dropped onto a 48-well plate to form droplets containing fibrinogen as a gelling agent. The droplet amount of the fibrinogen solution was 2 µL. The cell-containing solution of Test Example 1 containing thrombin was added dropwise to the droplets containing fibrinogen (reaction liquid). The injection amount of the cell-containing solution was 2 µL. That is, the volume ratio of the injection amount of the cell-containing solution to the droplet amount of the fibrinogen-containing droplet (reaction liquid) was 1:1. To promote gelation by the reaction between thrombin and fibrinogen, the droplets of the reaction liquid into which the cell-containing solution had been injected were cultured in a CO₂ incubator at 37°C. The gelling culture time was set to 30 minutes, 60 minutes, and 90 minutes, respectively. After the incubation was completed, 500 µL of the HCM medium was added to the culture substrate, and the culture was performed for 7 days.

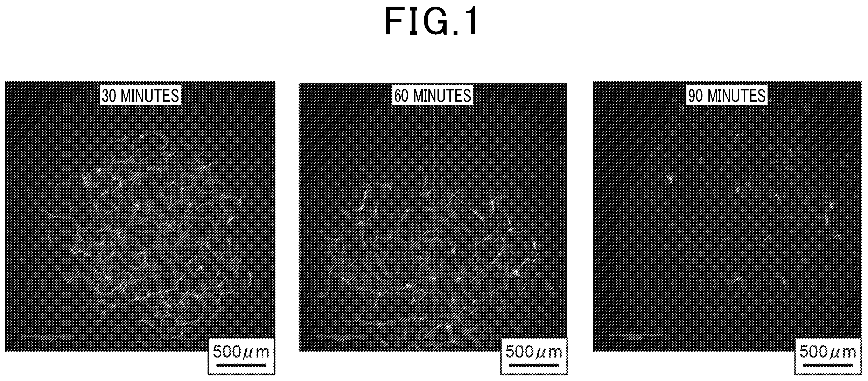

Fig. 1 shows the results of verifying the angiogenic ability at each gelling culture time through fluorescence observation of GFP-HUVECs. As seen from Fig. 1, the longer the incubation time, the lower the angiogenic ability.

### <Test Example 2>

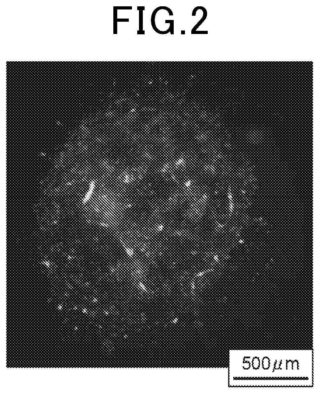

A cell structure was prepared in the same manner as in Test Example 1, except that the droplet amount of the fibrinogen solution in Test Example 1 was changed to 10 µL (the volume ratio of the injection amount of the cell-containing solution to the droplet amount of the droplet containing fibrinogen (reaction liquid) was changed to 5:1). Fig. 2 shows the result obtained when the gelling culture time was set to 30 minutes.

As seen from Fig. 2, when only one type of gelling agent was used and the droplet amount of the fibrinogen solution was increased, the injected cells spread over the entire droplet, causing the three-dimensional structure to collapse, and no vascular structure was observed.

### <Test Example 3>

A cell-containing solution of Test Example 3 containing cells and calcium ions as a gelling promoter was prepared in the same manner as in Test Example 1, except that a calcium aqueous solution prepared by dissolving calcium chloride in HCM medium to a concentration of 5 mM was used instead of the 20 U/ml thrombin solution dissolved in HCM medium.

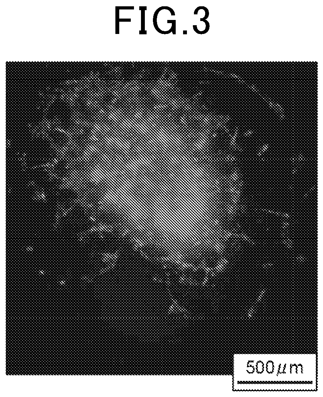

A 0.05 mass% sodium alginate aqueous solution prepared by dissolving sodium alginate in HCM medium at a concentration of 0.05 mass% was dropped onto a 48-well plate to form droplets containing sodium alginate as a gelling agent. The droplet amount of the solution containing sodium alginate was 10 µL. The cell-containing solution of Test Example 3 containing calcium ions was added dropwise to the droplets containing sodium alginate (reaction liquid). The injection amount of the cell-containing solution was 2 µL. That is, the volume ratio of the injection amount of the cell-containing solution to the droplet amount of the reaction liquid was 5:1. To promote gelation, the droplets of the reaction liquid into which the cell-containing solution had been injected were cultured in a CO₂ incubator at 37°C. The gelling culture time was set to 30 minutes. The subsequent steps were the same as in Test Example 1 to prepare a cell structure. Fig. 3 shows the result.

Fig. 3 shows that under other conditions in which only one type of gelling agent is used, the cells were excessively aggregated, which also prevented the formation of blood vessels.

### <Test Example 4>

A cell-containing solution of Test Example 4 containing thrombin and calcium ions as a gelling promoter was prepared in the same manner as in Test Example 1, except that a mixed solution containing thrombin and calcium ions was used instead of the 20 U/ml thrombin solution dissolved in HCM medium. The calcium ion concentration in the mixed solution was 5 mM relative to the total amount of the mixed solution (cell-containing solution). The thrombin concentration in the mixed solution was 20 U/ml relative to the total amount of the mixed solution (cell-containing solution).

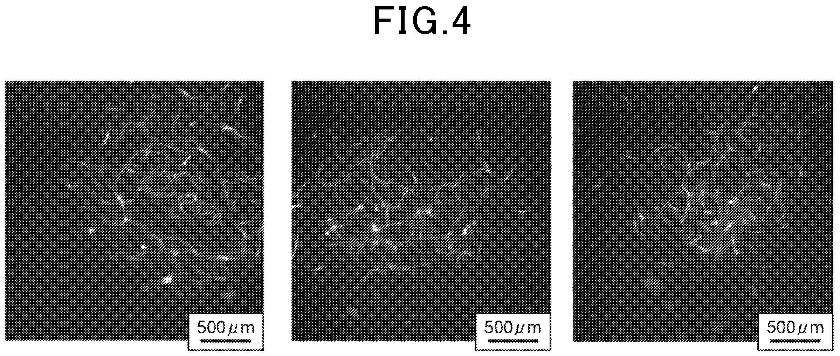

A mixed solution of sodium alginate and fibrinogen prepared by dissolving sodium alginate and fibrinogen in HCM medium was dropped onto a 48-well plate to form droplets containing sodium alginate and fibrinogen as a gelling agent. The content of the sodium alginate in the mixed solution was 0.05 mass% relative to the total amount of the mixed solution (reaction liquid). The fibrinogen concentration in the mixed solution was 5 mg/ml relative to the total amount of the mixed solution. The droplet amount of the solution containing sodium alginate and fibrinogen (reaction liquid) was 10 µL. The cell-containing solution of Test Example 4 containing thrombin and calcium ions was added dropwise to the droplets containing sodium alginate and fibrinogen (reaction liquid). The injection amount of the cell-containing solution was 2 µL. The volume ratio of the injection amount of the cell-containing solution to the droplet amount of the reaction liquid was 5:1. To promote gelation, the droplets of the reaction liquid into which the cell-containing solution had been injected were cultured in a CO₂ incubator at 37°C. The gelling culture time was set to 30 minutes, 60 minutes, and 90 minutes. The subsequent steps were the same as in Test Example 1 to prepare a cell structure. Fig. 4 shows the results.

Comparing the results shown in Fig. 4 with those shown in Fig. 1 shows that under the conditions of using a plurality of types of gelling agents, there is no significant change in the angiogenic ability even when the incubation time (gelling culture time) is increased. Using a single gelling agent to make the gel larger did not result in formation of blood vessels as shown in Fig. 2 and Fig. 3, whereas using a plurality of types of gelling agents resulted in formation of blood vessels as shown in Fig. 4. Therefore, using a plurality of types of gelling agents enables formation of blood vessels even when droplets are made larger. As shown in Fig. 4, the angiogenic ability of the cell structure produced by the method of Test Example 4 was not affected by the incubation time.

### <Test Example 5>

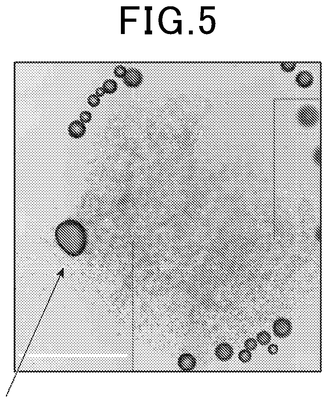

A cell structure was produced in the same manner as in Test Example 1, except that only hepatocytes were used. The gelling culture time was set to 30 minutes. Fig. 5 shows the result. The scale bar in the micrograph shown in Fig. 5 indicates 1 mm.

As seen from Fig. 5, when only one gelling agent (only fibrinogen) was used and the amount of gel was small, the gel was damaged by the impact when the culture medium was added, causing the cells to leak out. The arrow in Fig. 5 indicates the region where the gel is damaged and the cells are leaked out.

### <Test Example 6>

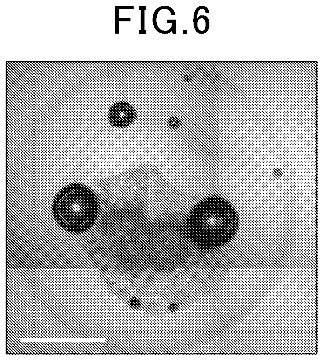

A tissue was produced in the same manner as in Test Example 5, except that 10 µL of a gelling agent composed of fibrinogen and sodium alginate was used and that the cell-containing solution contained 5 mM calcium ions, that is, in the same manner as in Test Example 4, except that only hepatocytes were used. Fig. 6 shows the result. The scale bar in the micrograph shown in Fig. 6 indicates 1 mm.

As seen from Fig. 6, using a plurality of types of gelling agents prevents tissue damage from occurring, and the cells are appropriately aggregated.

### <Test Example 7>

A cell-containing solution containing 20 U/ml thrombin and 5 mM calcium ions as a gelling promoter was prepared in the same manner as in Test Example 4, except that GFP-SEC in which GFP was introduced into hepatocytes (PXB Cells) and sinusoidal endothelial cells (SEC, manufactured by Sciencell) by a common genetic recombination method using lentivirus was used and that LX2 was not used.

A mixed solution of sodium alginate and fibrinogen prepared by dissolving sodium alginate and fibrinogen in HCM medium was dropped onto a 24-well transwell insert to form droplets containing sodium alginate and fibrinogen as a gelling agent. The content of the sodium alginate in the mixed solution was 0.05 mass% relative to the total amount of the mixed solution (reaction liquid). The fibrinogen concentration in the mixed solution was 5 mg/ml relative to the total amount of the mixed solution. The droplet amount of the solution containing sodium alginate and fibrinogen (reaction liquid) was 20 µL. The cell-containing solution of Test Example 4 containing thrombin and calcium ions was added dropwise to the droplets containing sodium alginate and fibrinogen (reaction liquid). The injection amount of the cell-containing solution was 2 µL. The volume ratio of the injection amount of the cell-containing solution to the droplet amount of the reaction liquid was 10:1. To promote gelation, the droplets of the reaction liquid into which the cell-containing solution had been injected were cultured in a CO₂ incubator at 37°C. The gelling culture time was set to 30 minutes. After the incubation was completed, 500 µL of the HCM medium was added to the culture substrate, and then the culture was performed for 14 days.

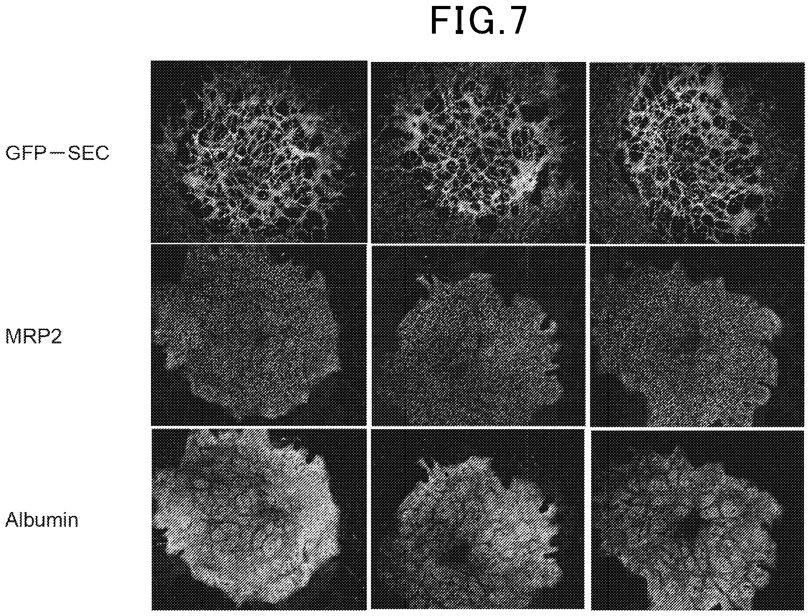

The GFP-SEC images (for three trials) in Fig. 7 are the results of verifying the angiogenic ability on Day 14 through fluorescence observation of GFP-SE. As seen from the GFP-SEC images in Fig. 7, the vascular network was fully formed and maintained even on Day 14, with the three-dimensional structure formed without collapse and no excessive aggregation occurring.

After culturing for 14 days, the cell structure was fixed with a 10% neutral buffered formalin solution. After treatment with mouse-derived anti-MRP2 antibody and rabbit-derived anti-albumin antibody, immunostaining with Alexa647-labeled anti-mouse IgG secondary antibody and Alexa546-labeled anti-rabbit IgG secondary antibody was performed, and the expression of MRP2 and albumin was evaluated using a confocal microscope. MRP2 is a transporter expressed on the bile canaliculi formed between hepatocytes having mature functions, and albumin is a protein that is produced within hepatocytes and serves as a marker for hepatocytes.

MRP2 and Albumin in Fig. 7 show images of cell structures stained with the respective antibodies. As seen from the images of Fig. 7, the hepatocytes were accumulated without excessive aggregation in the cell structure, and the formation of bile canaliculi indicated by the stained image of MRP2 was clearly and densely observed between the hepatocytes. The above results show that, when a cell structure is formed using sinusoidal endothelial cells by a method according to an aspect of the present invention, the three-dimensional structure is appropriately maintained and the vascular network is maintained without adversely affecting the characteristics of the hepatocytes.

## Claims

1. method for producing a cell structure having a vascular structure, the method comprising:
a gelation step of gelling a cell-containing solution containing cells including vascular endothelial cells to form a gel containing the cells; and
a culture step of culturing the cells in the gel, wherein the gel containing the cells is formed by bringing a plurality of gelling agents into contact with a gelling promoter.

2. The method according to claim 1, wherein
the cell-containing solution further contains an extracellular matrix component and a polyelectrolyte.

3. The method according to claim 2, wherein
the extracellular matrix component comprises collagen.

4. The method according to claim 2, wherein
the polyelectrolyte comprises heparin.

5. The method according to claim 1, wherein
the plurality of gelling agents comprise fibrinogen and sodium alginate.

6. The method according to claim 1, wherein
the gelling promoter comprises thrombin and calcium chloride.

7. The method according to any one of claims 1 to 6, wherein
the gelation step is a step of forming the gel by injecting the cell-containing solution containing the cells and the gelling promoter into a droplet of a reaction liquid containing the plurality of gelling agents.

8. The method according to claim 7, wherein
a volume ratio of a droplet amount of the reaction liquid to an injection amount of the cell-containing solution is 2 to 10.

9. The method according to any one of claims 2 to 4, further comprising, prior to the gelation step, a step of mixing the cells, the extracellular matrix component and the polyelectrolyte to obtain a mixed solution.

10. The method according to claim 9, further comprising, prior to the gelation step, a step of centrifuging the mixed solution to obtain a precipitate containing the cells, the extracellular matrix component and the polyelectrolyte.

11. The method according to any one of claims 2 to 4, wherein
a concentration of the polyelectrolyte is greater than 0 mg/mL and 1.5 mg/mL or less relative to a total amount of the cell-containing solution.

12. The method according to any one of claims 2 to 4, wherein
a concentration of the extracellular matrix component is greater than 0 mg/mL and 1.5 mg/mL or less relative to a total amount of the cell-containing solution.

13. The method according to any one of claims 1 to 6, wherein
the cells further comprise hepatocytes.

14. The method according to any one of claims 1 to 6, wherein
the cells further comprise hepatic stellate cells.

15. The method according to any one of claims 1 to 6, wherein
a ratio of the number of vascular endothelial cells to the total number of cells is 5% or greater and 50% or less.

16. A method for producing a cell structure having a vascular structure, the method comprising:
a step of mixing cells including vascular endothelial cells, an extracellular matrix component and a polyelectrolyte to obtain a mixed solution;
a step of centrifuging the mixed solution to obtain a precipitate containing the cells, the extracellular matrix component and the polyelectrolyte;
a step of obtaining a cell-containing solution containing the precipitate and a gelling promoter;
a step of forming a gel by injecting the cell-containing solution into a droplet of a reaction liquid containing a plurality of gelling agents; and
a culture step of culturing the cells in the gel, wherein
a concentration of the polyelectrolyte is greater than 0 mg/mL and 1.5 mg/mL or less relative to a total amount of the cell-containing solution,
a concentration of the extracellular matrix component is greater than 0 mg/mL and 1.5 mg/mL or less relative to a total amount of the cell-containing solution,
the plurality of gelling agents include fibrinogen and sodium alginate,
a content of the fibrinogen is 1 mg/ml to 10 mg/ml relative to a total amount of the reaction liquid,
a content of the sodium alginate is 0.01 mass% to 2 mass% relative to a total amount of the reaction liquid,
the gelling promoter includes thrombin and calcium ions,
a content of the thrombin is 10 unit/ml to 30 unit/ml relative to a total amount of the cell-containing solution,
a content of the calcium ions is 1 mM to 10 mM relative to a total amount of the cell-containing solution, and
a volume ratio of a droplet amount of the reaction liquid to an injection amount of the cell-containing solution is 2 to 10.

17. A method for producing a cell structure having a vascular structure, the method comprising:
a step of mixing cells including vascular endothelial cells, an extracellular matrix component and a polyelectrolyte to obtain a mixed solution;
a step of centrifuging the mixed solution to obtain a precipitate containing the cells, the extracellular matrix component and the polyelectrolyte;
a step of obtaining a cell-containing solution containing the precipitate and a gelling promoter;
a step of forming a gel by injecting the cell-containing solution into a droplet of a reaction liquid containing a plurality of gelling agents; and
a culture step of culturing the cells in the gel, wherein
the polyelectrolyte includes heparin,
a concentration of the polyelectrolyte is greater than 0 mg/mL and 1.5 mg/mL or less relative to a total amount of the cell-containing solution,
the extracellular matrix component includes collagen,
a concentration of the extracellular matrix component is greater than 0 mg/mL and 1.5 mg/mL or less relative to a total amount of the cell-containing solution,
the plurality of gelling agents include fibrinogen and sodium alginate, and
the gelling promoter includes thrombin and calcium ions.
